# EUROPEAN PATENT APPLICATION

(11) **EP 2 070 541 A1**
(43) Date of publication of application: **17.06.2009**
(21) Application number: 07828859.4
(22) Date of filing: 21.09.2007
(51) Int. Cl.: A61K 35/02, A23K 1/175, A23L 1/30, A61L 27/00, A61P 1/02, A61P 19/00

(54) **PROMOTER OF HARD TISSUE REGENERATION**

(30) Priority: 21.09.2006 JP 2006256374
(71) Applicant: Oguro, Toshiki, Tokyo 141-0001 (JP)
(72) Inventor: Oguro, Toshiki, Tokyo 141-0001 (JP)
(74) Representative: Jones, Helen M.M.
(86) International application number: PCT/JP2007/069118
(87) International publication number: WO 2008/035816

(57) **Abstract**

The object of the present invention is to provide a hard-tissue regeneration promoter, a pharmaceutical composition, a food composition, a food, etc., useful for regeneration of an impaired or defective hard tissue, which can be prepared in low production and labor costs. That is, regeneration of a hard tissue in an impaired or defective area can be promoted by causing such an agent or composition to contain diatomaceous earth and administering the resulting agent/composition beneath the periosteum in the vicinity of the impaired or defective hard tissue or orally administering the same.

## Description

### CROSS REFERENCE TO RELATED APPLICATIONS

This application claims the benefit of priority to Japanese Patent Application No. 2006-256374, filed on September 21, 2006, which is incorporated herein by reference.

### TECHNICAL FIELD

The present invention relates to agents, pharmaceutical compositions, food compositions, foods, animal feeds, etc., useful for regeneration of hard tissues.

### BACKGROUND ART

In order to restore impaired or defective areas of hard tissues, conventionally, methods for filling an impaired or defective area with filling materials such as artificial hydroxyapatite, cow bone dried powder, silica glass powder, and Portland cement (U.S. Patent No. 5769638); a method for forming a bone tissue by inserting into an impaired area or a defective area a 300 to 360 µm granular material obtained by dissolving and then pulverizing the mixture of SiO₂ (45% by weight), CaO (24.5% by weight), Na₂O (24.5 % by weight), and P₂O₅ (6% by weight) (Japanese Laid-Open Application No.1991-136664); an agent for promoting bone regeneration (Japanese Laid-Open Application No. 2003-55237), etc. have been developed.

### DISCLOSURE OF THE INVENTION

### PROBLEMS TO BE SOLVED BY THE INVENTION

The object of the present invention is to provide hard-tissue regeneration promoters, pharmaceutical compositions, food compositions, foods, animal feeds, etc., useful for regeneration of an impaired or defective hard tissue, which can be prepared in low cost of production and labor.

### MEANS FOR SOLVING THE PROBLEMS

As shown in the Examples, the inventors of the present application found that regeneration of a defective hard issue can be promoted by administering diatomaceous earth under the periosteum in the vicinity of the area having the defective hard tissue or orally administering diatomaceous earth, and have thus accomplished the present invention.

Namely, the hard-tissue regeneration promoter according to the present invention contains diatomaceous earth as an active ingredient. The aforementioned hard tissue is exemplified by a hard bone, a reparative dentin, etc., the hard bone being exemplified by an alveolar bone, etc. The hard-tissue regeneration promoter according to the present invention may be administered or filled beneath the periosteum, may be administered into the defective medullary cavity associated with tooth extraction or the like or into the medullary cavity in the vicinity of a fractured site, or may be administered orally.

Further, the pharmaceutical composition according to the present invention contains diatomaceous earth as an active ingredient. The pharmaceutical composition according to the present invention may be a preparation to be administered beneath the periosteum, may be a preparation to be administered into the defective medullary cavity associated with tooth extraction or the like or into the medullary cavity in the vicinity of a fractured site, or may be a preparation to be administered orally.

The food composition according to the present invention contains diatomaceous earth.

The food according to the present invention contains diatomaceous earth.

The animal feed according to the present invention contains diatomaceous earth.

As used herein, the term "diatomaceous earth" refers to a deposit composed of the remains of diatom, including marine diatomaceous earth including diatomaceous ooze, which is a deposit of marine diatom and freshwater diatomaceous earth, which is a deposit of freshwater diatom. The diatomaceous earth contained in the agent, pharmaceutical composition, food composition, food, animal feed, etc. according to the present invention may be either a burned product which is burned at a high temperature or a dried product dried with natural air.

### BRIEF DESCRIPTION OF THE DRAWINGS

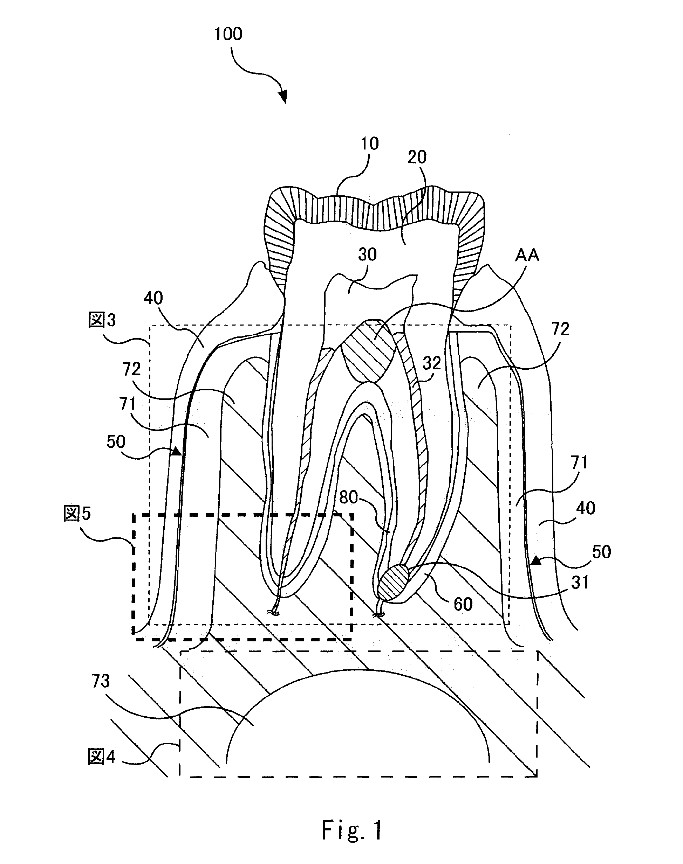

FIG. 1 shows the tooth structure explained as one embodiment according to the present invention.

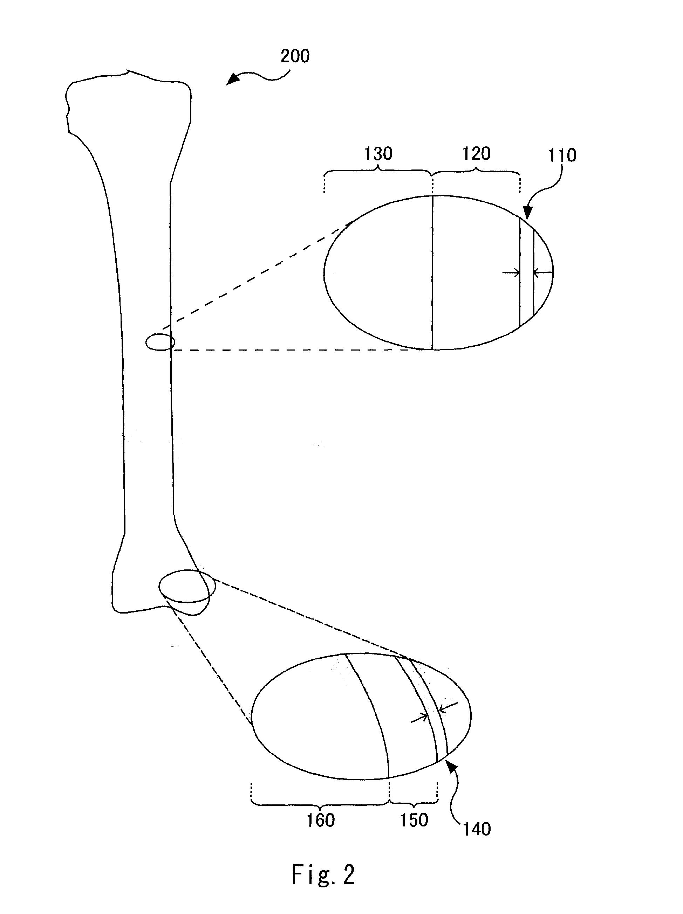

FIG. 2 shows the bone structure explained as one embodiment according to the present invention.

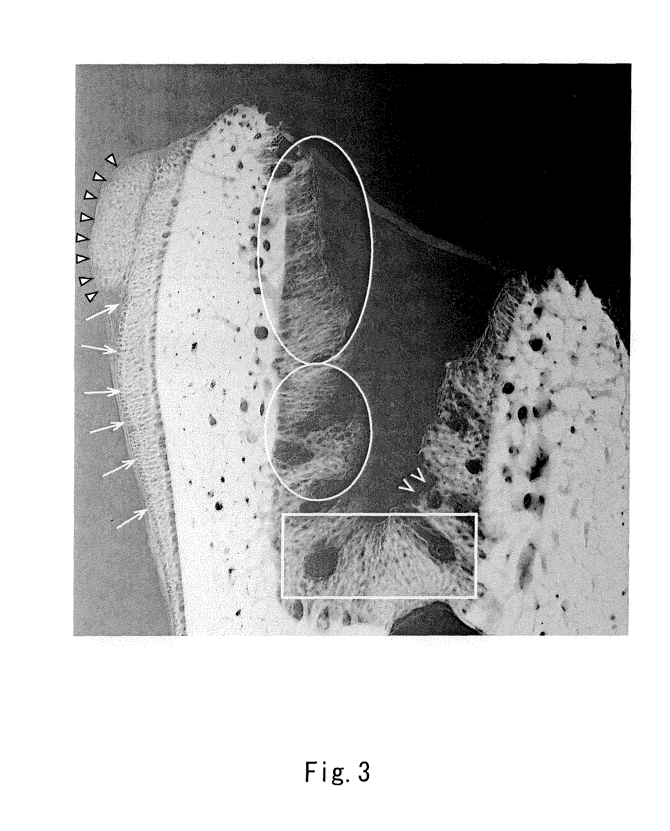

FIG. 3 shows the result obtained by observing the vicinity of the area where a tooth was extracted and the vicinity of the site where diatomaceous earth was administered using a micro-focus X-ray enlarging observation system after administering the diatomaceous earth into the periosteum in one example according to the present invention.

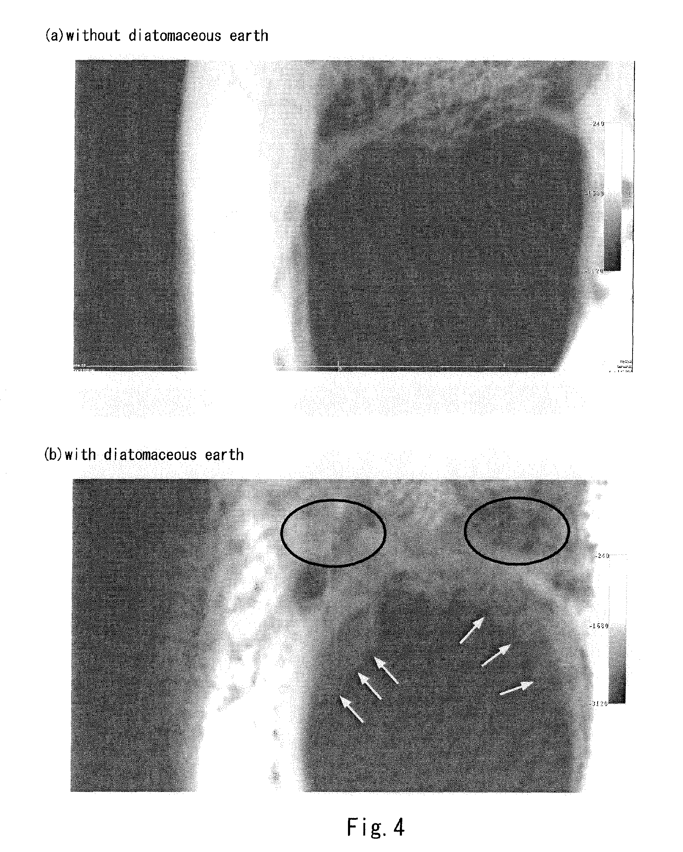

FIG. 4 shows the result obtained by observing the vicinity of the area where a tooth was extracted and the lower alveolar part using a micro-focus X-ray enlarging observation system after administering the diatomaceous earth into the periosteum in one example according to the present invention.

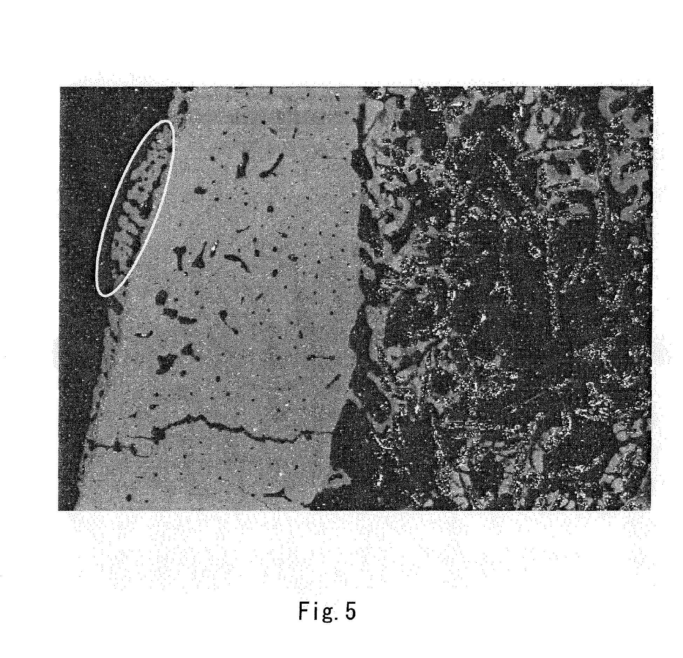

FIG. 5 shows the result of the verification that regeneration and healing of a bone after tooth extraction or after perforation of a basal bone for implant placement are promoted by oral administration of diatomaceous earth, using the element mapping method of calcium and diatomaceous earth-derived silicon by EPMA in one example according to the present invention.

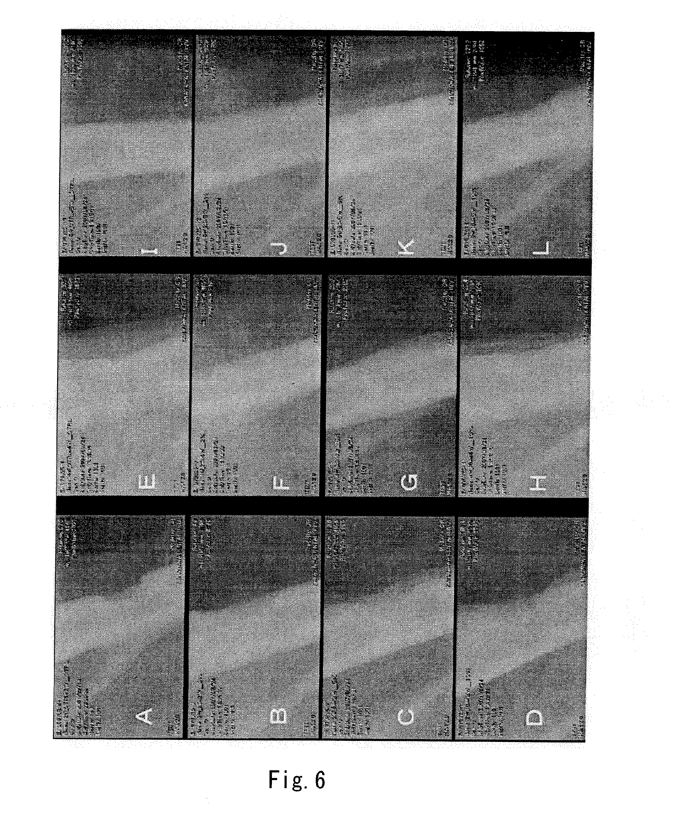

FIG. 6 is a photograph showing the result obtained by observing that bone regeneration is promoted in a lower limb bone which has been artificially perforated by oral administration of diatomaceous earth, using a micro-focus X-ray microscopic observation system, in one example according to the present invention.

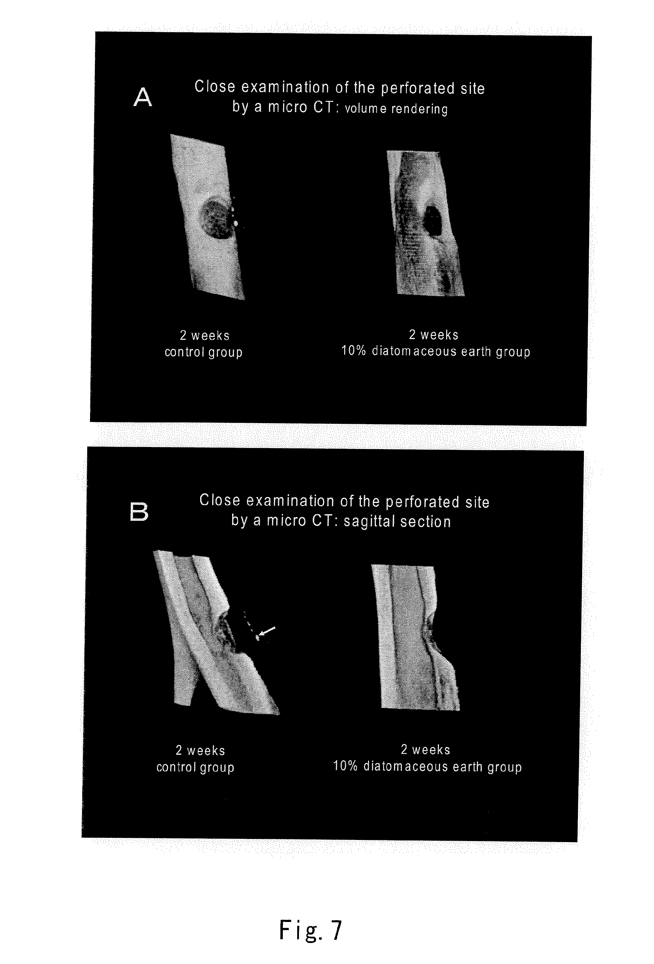

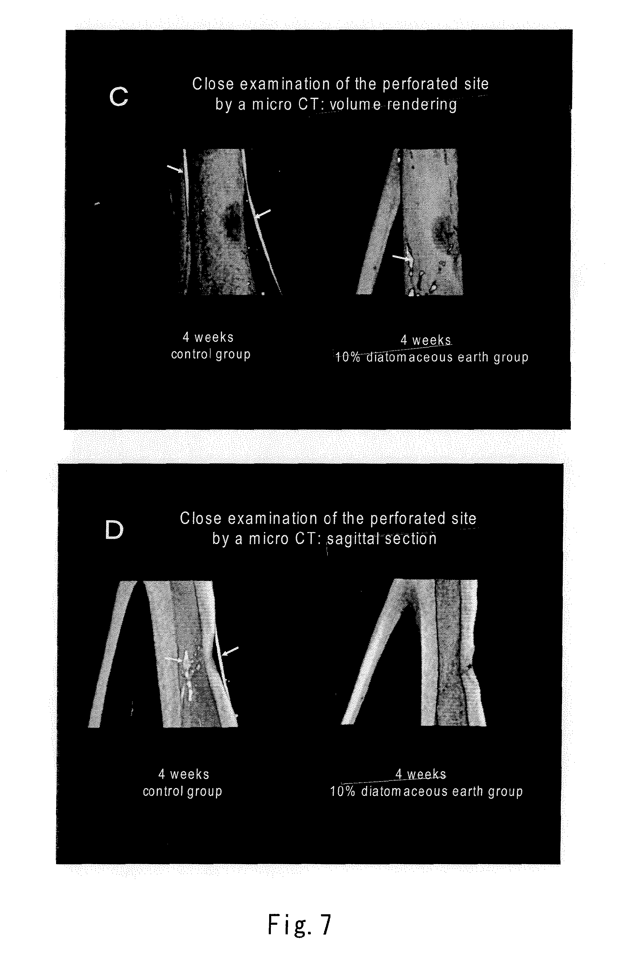

FIG. 7 is a photograph showing the result obtained by observing 3-dimensional microscopically that bone regeneration is promoted in a lower limb bone which has been artificially perforated by oral administration of diatomaceous earth, using a micro CT system, in one example according to the present invention. It should be noted that the linear high contrast shadows (indicated by white arrow heads) recognized near the bone or within the medullary cavity in each of the images are associated with angiography and not related to observation of bone regeneration.

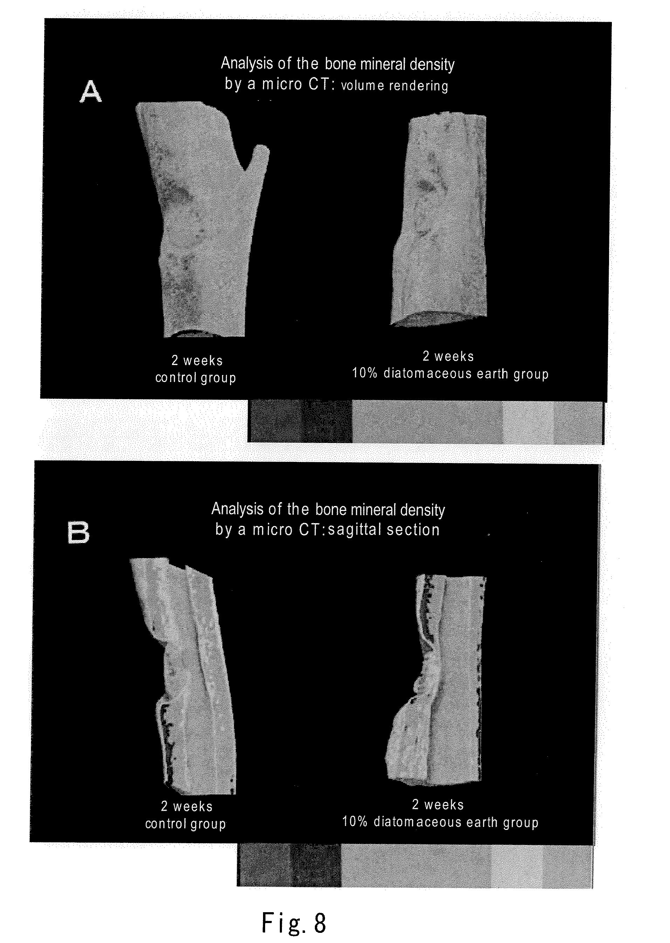

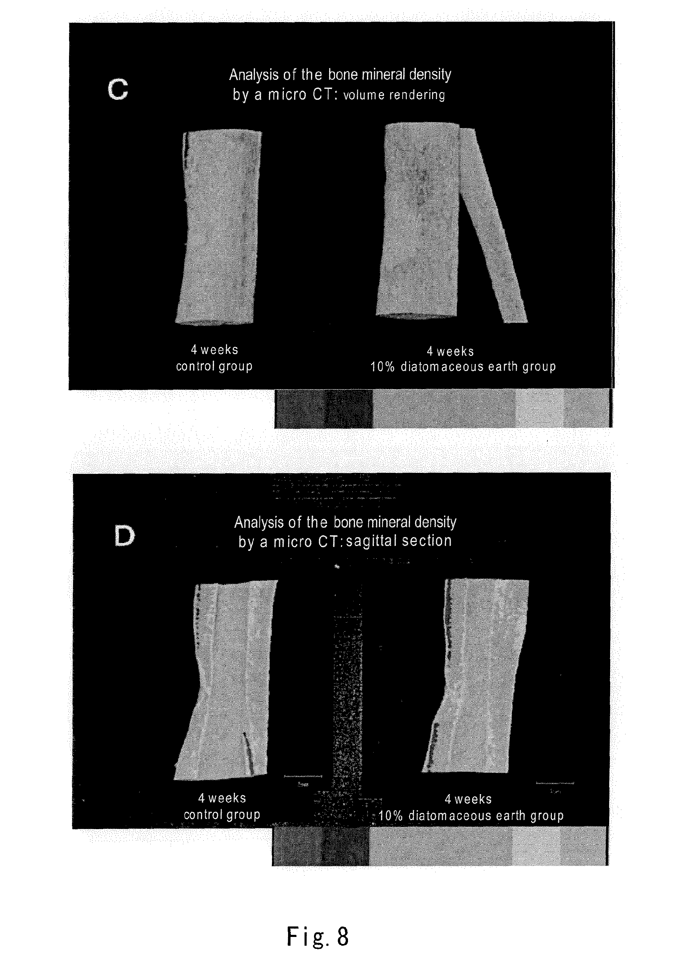

FIG. 8 is a photograph showing the result obtained by the quantitative analysis of the bone mineral density based on the X-ray microscopic image data shown in FIG. 7 in one example according to the present invention.

### BEST MODE FOR CARRYING OUT THE INVENTION

Embodiments of the present invention accomplished based on the above-described findings are hereinafter described in detail by giving Examples. When using commercial apparatuses, unless otherwise explained, protocols attached to them are used.

The object, characteristics, and advantages of the present invention as well as the idea thereof will be apparent to those skilled in the art from the descriptions given herein. It should be understood that the embodiments and specific examples of the invention described herein below are to be taken as preferred examples of the present invention. These descriptions are only for illustrative and explanatory purposes and are not intended to limit the invention to these embodiments or examples. It is further apparent to those skilled in the art that various changes and modifications may be made based on the descriptions given herein within the intent and scope of the present invention disclosed herein.

### == Pharmacological effect of diatomaceous earth ==

As shown in FIG. 1, a tooth 100 consists of an enamel 10, a dentine 20, a cementum 80, a pulp 30, etc., with components such as a gingiva (gum) 40, a periodontium 60 and a cortical bone (compact bone) 71, cancellous bone 72, etc. and is supported by periodontal tissues, such as alveolar bone which is a hard tissue.

As will be shown in Example 1, after a tooth had been extracted in a dog, the gingiva 40 adhering to an alveolar bone was removed together with the periosteum 50 and diatomaceous earth was filled beneath the periosteum 50 covering the surface of the alveolar bone. It was found that regeneration of a new bone was promoted in the defective cancellous bone 72, on the cortical bone 71 near the site where diatomaceous earth had been administered, at the bottom of the medullary cavity 73 near the defective region (alveolar bone) of the tooth 100, and above the medullary cavity 73. Further, as will be shown in Example 2, it was indicated that regeneration of a new bone was similarly promoted on the cortical bone 71 and the defective region of the tooth 100 (cancellous bone 72 in the alveolar region)by orally administering diatomaceous earth to a dog in which the inside of the alveolar bone had been smoothly drilled after a tooth had been extracted. These findings indicated that diatomaceous earth is useful in promoting regeneration of hard tissues in impaired or defective areas resulting from osteoclasis, absorption or fracture, due to tooth extraction, embedeling and fixation of an implant, toxin of the bacteria associated with endodontal or periodontal disease, infection, bone deletion expediently performed during an operation, etc.

The shaft of a hard bone, which is a hard tissue, such as a long bone 200 of the humerus, femur, etc., as shown in FIG. 2, consists of a periosteum 110, a cortical bone 120, a bone marrow 130, etc., the end portion of the bone 200 being composed of a periosteum 140, a cortical bone 150, a spongin 160, bone marrow, etc. When such a hard bone becomes partly impaired or defective due to fracture, implant embedding and fixation, bacterial toxin, suppuration, etc., the hard tissue can be regenerated in the impaired/defective area by administering diatomaceous earth beneath the periosteum 110 and/or the periosteum 140 in the vicinity of the impaired/defective area, administering diatomaceous earth into the medullary cavity, or orally administering diatomaceous earth. (The example of regeneration of a lower limb bone by oral administration will be shown in Example 3.) Therefore, diatomaceous earth is useful in promoting the regeneration of hard tissues in the areas which have become impaired or defective due to fracture, implant embedding, bacterial toxin, suppuration, etc.

Examples of the hard tissue in which regeneration can be promoted by using diatomaceous earth include hard bones (alveolar bones, jawbones, femurs, humeri, neck bones, radius, ulna, vertebra, etc.), reparative dentin of the tooth 100, and the like. The diatomaceous earth to be used is not particularly limited as long as it is a fossilized diatome, such as marine diatomaceous earth and freshwater diatomaceous earth, or a burned product or a dried product of diatomaceous earth.

### == The agent, pharmaceutical composition, food composition, food, etc., according to the present invention ==

As described above, diatomaceous earth, which promotes regeneration of impaired or defective hard tissues, is useful as pharmaceutical or food compositions for human patients in whom impaired or defective hard tissues are required to be regenerated and as feed compositions for animal patients in which impaired or defective hard tissues are required to be regenerated. Further, the agent containing diatomaceous earth as an active ingredient is useful as an agent which promotes regeneration of impaired or defective hard tissues. The food containing diatomaceous earth is useful as a functional food, a food for specified health uses, etc. for human patients in whom regeneration of impaired or defective hard tissues is required. The animal feed containing diatomaceous earth is useful for animal patients in which regeneration of impaired or defective hard tissues is required. Moreover, being a naturally-occurring material, diatomaceous earth is available at low prices and in large quantities. Therefore, the agents, pharmaceutical compositions, food compositions, foods, etc. according to the present invention can be provided in a low production and labor costs.

The agent according to the present invention agent is not particularly limited as long as it contains diatomaceous earth as an active ingredient. It may further contain a pharmacologically acceptable formulation additive (e.g., an excipient, a pH adjuster, a binder, a lubricant, a disintegrator, a corrigent, a solvent, a stabilizer, a sugar coating agent, a preservative, a buffer, a suspending agent, an emulsifier, an aromatic, a solubilizing agent, a colorant, a viscous agent, etc.) depending on the body part at which it is used, the purpose of its use, or the mode of its administration. In addition, it may further contain an agent for promoting restoration and/or regeneration of periodontal tissues, preventing bacterial infection or suppressing inflammation, which is exemplified by a polyphosphate such as sodium polyphosphate and alginic acid as a wound dressing material.

In addition, the agent according to the present invention may be administered, for example, orally to human or non-human vertebrates. Alternatively, it may be administered into the medullary cavity or beneath the periosteum 50/110/140, etc. For oral administration of the agent according to the present invention, it may be prepared into tablets, capsules, granules, powder, syrups, pills, etc. For administration beneath the periosteum 50/110/140, it may be prepared into injections or the like. These preparations can be produced by the conventional methods using above-mentioned formulation additives.

Moreover, the food according to the present invention may be any kind of food as long as it contains diatomaceous earth. Also, besides diatomaceous earth, it may further contain an existing food additive (s) such as an antioxidant (e.g., vitamin A, C, E, K, polyphenol, etc.), a sweetener (e.g., aspartame, xylitol, etc.), a preservative (e.g., potassium sorbate, sodium benzoate, etc.), an antioxidant (e.g., sodium sulfite, L-ascorbic acid sodium, etc.), a coloring agent (turmeric pigment, paprika pigment, etc.), a seasoning (amino acid, meat extract, etc.), an acidulant (e.g., citric acid, malic acid, etc.), and/or the like.

The food according to the present invention can be used in the form of, but not limited to, for example, supplements (in capsule, tablet, or granular form), noodles (e.g., Japanese noodles etc.), yogurt, hard sweets (e.g., candies, drops, etc.), seasoned powders. It should be noted that the food according to the present invention can be in any form such as solid, powder, liquid, fluid, cream, gel, etc. In addition, the food according to the present invention is designed in principle for healthy people, but may be digested by those patients in whom regeneration of impaired or defective hare tissues is required.

The animal feed according to the present invention is not limited as long as it contains diatomaceous earth and may further contain, besides diatomaceous earth, an existing feed additive, such as an antioxidant, an antifungal agent, a binder, an emulsifier, a regulator, an amino acid, a vitamin, a mineral, pigment, synthetic antimicrobials, antibiotics, a flavoring, a taste-improving agent, an enzyme, a probiotic agent, etc.

### == Uses of the agent, pharmaceutical composition, food, etc. according to the present invention ==

The hard-tissue regeneration promoter containing diatomaceous earth as an active ingredient may be administered anywhere in the body of the patient having a impaired or defective hard tissue, but it is preferably administered directly to the impaired or defective area or to the vicinity of the area in which regeneration of a hard tissue is required. For example, regeneration of hard bone can be promoted by administration beneath the periosteum 50/110/140, located nearest to the bone required to be regenerated, or to a perforated area in pulp cavity AA, a root tip 31, or the root canal 32 of a tooth 100. Further, when the tooth 100 becomes impaired or defective, regeneration of reparative dentin is promoted by filling the dentine 20 of the impaired or defective tooth 100 or other such area with the aforementioned promoter. In particular, when the tooth 100 becomes impaired or defective as deep as the tooth pulp 30, the dentine 20 of the impaired or defective tooth 100 or other point may be filled so that the tooth pulp 30 will not be exposed. Examples of the mode of administration includes, but not limited to, infusion, spreading, restoration, etc. Alternatively, the hard-tissue regeneration promoter may be orally administered to a patient who has the impaired or defective hard tissue.

Moreover, intake of diatomaceous earth may be started, as a pharmaceutical composition or a food containing diatomaceous earth as an active ingredient, even before a hard tissue becomes impaired or defective so that the effect of promoting hard-tissue regeneration can be exerted immediately.

### EXAMPLES

Hereinafter, the present invention will be specifically explained using examples, which are provided solely for purposes of illustration and are not to be construed to limit the scope of the present invention to these examples.

### EXAMPLE 1

An alveolar bone defect was created in dogs, and, diatomaceous earth was administered beneath the periosteum to promote new bone regeneration at the defected site.

The gingiva and periosteum of the dogs were scraped with a raspatory when the defective tooth was extracted. 0.2 g of diatomaceous earth (dried product; Chuo Kasei Co., Ltd. and Chuo Silica Co., Ltd.), dissolved in distilled water to form a paste, was filled beneath the periosteum. After one month, using a micro-focus X-ray inspection system, P70 II (focal spot size: 10 µm x 10 µm), manufactured by Pony Industry Co., Ltd., enlarged X-ray images of the vicinity of the site where diatomaceous earth had been injected were obtained using a film (FIG. 3) or an imaging plate (FIG. 4) by placing a photo-film manufactured by Eastman Kodak Co., a cassette containing an HR type imaging plate manufactured by Fuj ifilm Medical Co., Ltd., respectively, with 3 x geometric enlargement ratio under the exposure setting of 30 kV, 90 µA for 15 min for the film and 30 sec for the imaging plate. The image data recorded on the imaging plate were read and observed using a Fuji computed radiography (FCR) system (FCR 5000MA, which is a digital X-ray imaging diagnostic system manufactured by Fuji Photo Film Co., Ltd.) (refer to FIG. 1 for the area observed). As the control, the dogs to which diatomaceous earth had not been administered were observed using the micro-focus X-ray enlarging observation system. The results are shown in FIGs. 3 and 4. The arrowheads in FIG. 3 show the area where diatomaceous earth has been administered.

In the dogs to which diatomaceous earth had not been administered, new bone formation was observed only to a slight extent on the inner wall of the medullary cavity below the extraction socket (FIG. 4) as well as on the subperiosteal cortical bone (compact bone). In contrast, as shown in FIGs. 3 and 4, in the dogs to which diatomaceous earth had been administered, formation of developing new bones was observed at the bottom of the medullary cavity of the extraction socket (boxed in FIG. 3 and circled in FIG 4) ; on the side wall of the medullary cavity (refer to the circled area in FIG. 3); on the subperiosteal cortical bone near the area where diatomaceous earth has been administered (arrowed in FIG. 3) ; and in the lower alveolar part (arrowed in FIG. 4) and formation of cancellous bone was also observed (indicated by V in FIG. 3) in the medullary cavity of the extraction socket. Time course observation of these areas indicated that new bones formed in the order of "on the subperiosteal cortical bone → the bottom of the medullary cavity and the side wall of the medullary cavity → the cancellous bone inside the medullary cavity. Thus, diatomaceous earth promotes osteogenesis by being administered beneath the periosteum. Further, since administration of diatomaceous earth beneath the periosteum leads to new bone formation in the medullary cavity, it was found that the direct administration of diatomaceous earth into the medullary cavity also promotes osteogenesis.

### EXAMPLE 2

Next, an alveolar bone defect was created in dogs, and, diatomaceous earth was orally administered to promote new bone regeneration.

After tooth extraction, the inside of the alveolar bone of the dogs was drilled smoothly. Then, the dogs were administered an oral dose of 8 to 20 g of diatomaceous earth together with their food, which corresponded to 2 to 5% of their daily food intake. After one month, by preparing sections, the new bone tissues near the defective alveolar bone were examined by calcium and silicon element mapping using an EPMA (Electron Probe Micro Analyzer; Shimadzu EPMA8705, acceleration voltage 2 kV, beam current 15 nA, MAP 512 x 512 point), (refer to FIG. 1 for areas observed). The results are shown in FIG. 5.

As shown in FIG. 5, silicon derived from diatomaceous earth (white powder-like signal in FIG. 5) was distributed inside the bones, and in its surrounding are (the defective area in the cancellous bone inside the alveolar bone) new bone formation (gray area of the defective bone in FIG. 5) was observed. Moreover, new bone formation was also recognized on the cortical bone 71 (circled in Fig. 5). These findings indicate that when diatomaceous earth was orally administered, silicon reached down to the defective area of a bone and formed new bone there. Therefore, it was concluded that diatomaceous earth is effective not only in administration into the periosteum and administration into the medullary cavity but also in oral administration.

### EXAMPLE 3

Further, lower limb bones of rats were artificially perforated and diatomaceous earth was orally administered to promote new bone regeneration in the perforated area.

### [Treatment of samples]

Nine-week-old female BrlHan:WIST@Jcl (GALAS) rats (CLEA Japan) were used for this experiment. After acclimation, the rats were divided into groups two days before the scheduled date of treatment.

On the day of the treatment, lower limb bones (tibiae) of the rats were exposed such that damage to the periosteum would be minimal, and a hole (slightly more than about 2 mm in diameter and about 3 mm in depth) was made under irrigation with physiological saline using a dental implant drill (round bur). In light of the depth, it was confirmed that the head of the round bur went into the bone marrow through the cortical bone. Then, the rats were maintained on oral intake of diatomaceous earth which had been mixed with the standard laboratory chow CE-2 at weight ratios of 2%, 5%, and 10% and sterilized with electron beam irradiation.

Two weeks, four weeks, and five weeks after perforation, the right and left lower limbs were resected from each rat. After the right and left limbs were fixed separately in formalin, and stored in cool conditions prior to treatment. For comparison, rats in the control group underwent perforation operation in the same manner but fed only the standard laboratory chow cE-2, without diatomaceous earth, which was sterilized with electron beam radiation. The rats were also maintained, treated, and observed in the same manner.

### [Methods for preparing and observing samples]

### (1) X-ray microscopic observation using the micro-focus X-ray microscopic observation system

First, to observe nondestructively the sites of perforated wounds, a cassette containing an HR type imaging plate manufactured by Fujifilm Medical Co., Ltd. was placed with 3 x geometric enlargement ratio in a micro-focus X-ray inspection system, P70 II (focal spot size: 10 µm x 10 µm) manufactured by Pony Industry Co., Ltd., enlarged X-ray images of a mass of the bones from which the soft tissue had not been removed were projected under the exposure setting of 30 kV, 90 µA for 10 sec. Then, the image data recorded on the imaging plate was scanned by a digital X-ray imaging diagnostic system FCR 5000MA manufactured by Fuji Photo Film Co., Ltd. and x-ray microscopic images of each sample were obtained. Referring to FIG. 6, the results obtained by the observation are described in detail hereinbelow.

### (2) X-ray stereomicroscopic observation using a micro CT system

The 3-dimensional structure of the wound healing sites at 2 weeks and 4 weeks after perforation were closely examined using MCT-CB100MF manufactured by Hitachi Medical Corp. Each sample, fixed with a cellophane tape in the semi-cylindrical tube formed by longitudinally splitting a polypropylene centrifuge tube, was secured on the scan table. Exposure setting was 60 kV, 100 µA for 2 min. Referring to FIG. 7, the results obtained by the observation are described in detail hereinbelow.

### (3) Quantitative analysis of bone mineral density in the X-ray microscopic image data with a micro CT system

Time course relative changes of bone mineral density in the microscopic CT image data were compared using TRI 3D-BON-BMD manufactured by Ratoc System Engineering Co., Ltd. The bone mineral density was standardized using, as a substitute for the phantom for bone mineral density determination, the cross section of the polypropylene centrifuge tube on the scan table and the cortical bone area distal to the perforated wound. Referring to FIG. 8, the results of the analysis are described in detail hereinbelow. It should be noted that, in FIG.8, the color map is set such that the region(s) corresponding to the red color in the left side of the color bar represents the highest bone mineral density and that the more toward the red in the right side of the color bar the relatively lower bone mineral density.

### [Result]

### (1) X-ray microscopic observation using the micro-focus X-ray microscopic observation system

At 2 weeks after perforation (FIG. 6 A to D): In the individual rats in the control group (FIG. 6A), a semicircular depression having a diameter close to that of the perforated hole was clearly observed, whereas in the diatomaceous earth-administered groups (FIG. 6 B:2%, C:5%, D:10%) callus-like radiopaque region was observed in the semicircular depression; and as the concentration of the diatomaceous earth administered increased, the area of the radiopaque region increased in proportion to the concentration. Furthermore, in the diatomaceous earth-administered groups, remarkable reparative hypertrophy of the cortical bone was recognized in the bone marrow side of the bottom of the semicircular depression.

At 4 weeks after perforation (FIG. 6 E to H): In the individual rats in the control group (FIG. 6A) the semicircular depression became shallow and recovered enough to appear as a dish-like depression. On the other hand, in the diatomaceous earth-administered groups (FIG. 6 B:2%, C:5%, D:10%) such a dish-like depression was hardly observed; and as the concentration of the diatomaceous earth administered increased, the thickness of the cortical bone increased in proportion to the concentration. It should be noted that in the diatomaceous earth-administered groups, the radiolucency due to the bone regeneration which filled the site corresponding to a semicircular depression that had been observed two weeks after the perforation was recovered to the same degree as the surrounding areas; and at the bone marrow side near the area corresponding to the bottom of the depression site, a slight reparative hypertrophy of the cortical bone was observed.

At 5 weeks after perforation (FIGs. 6 I to L): In all the rats in the control group (FIG. 6I) as well as the diatomaceous earth-administered groups (FIG. 6 J:2%, K:5%, L:10%), the semicircular depression area disappeared and the cortical bone was recovered to almost normal morphology. It should be noted that in the diatomaceous earth groups, the reparative hypertrophy at the bone marrow side of the cortical bone observed 4 weeks after perforation still rudimentarily remained.

Thus, promotion of bone regeneration in the perforation area was observed by orally administering diatomaceous earth to the rats whose lower limb bones had been perforated.

### (2) X-ray stereomicroscopic observation using a micro CT system

### At 2 weeks after the perforation:

Volume-rendering image (FIG. 7A): In the control rats, a semicircular depression having a diameter close to that of the perforated hole was still clearly observed, whereas in the 10% diatomaceous earth-administered group, an obvious decrease in the diameter of the semicircular perforation area was observed and thus closure and healing of the wound caused by the perforation has been progressed. On the other hand, in the 10% diatomaceous earth-administered group, the radiolucency of the surrounding cortical bone increased.

Sagittally sectional image (FIG. 7B) : In the control rats, a semicircular depression having a diameter close to that of the perforated hole was clearly observed. Moreover, the bottom of the depression area was still broadly communicating with the medullary cavity; closure and healing of the perforation wound or regeneration of the cortical bone was hardly observed. In contrast, in the 10% diatomaceous earth-administered group, an obvious decrease in the depth of the semicircular depression was observed, the communication of the bottom of the depression area with the medullary cavity was narrowed, and thus closure and healing of the perforation wound as well as a sign of regeneration of the cortical bone was observed. Meanwhile, inside the neighboring cortical bone, lacunae were observed in the lamellar bone, which corresponded with the finding of an increase in radiolucency observed in the rendering image. This was probably because, in the diatomaceous earth-administered groups, at this stage, early mineralization in the wound and subsequent bone substitution resulting from calcification are vigorously promoted by diatomaceous earth, thereby coordinately activating absorption and dissolution of calcific component of bones as well as its addition to the wound in the vicinity.

### At 4 weeks after the perforation:

Volume-rendering image (FIG. 7C): Both in the control groups and diatomaceous earth groups, the semicircular depression became shallow and recovered enough to appear as a dish-like form. However, in the control rats, a large remaining perforation was clearly observed in the center, whereas in the diatomaceous earth-administered groups, only an extremely small remaining perforation was observed in the center.

Sagittally sectional image (FIG. 7D) : In the control rats, although the semicircular depression became shallow, the thickness at the periosteum side of the cortical bone was insufficient and the thicknesses of all its layers were also insufficient. Meanwhile, in the diatomaceous earth-administered groups, a semilunar bone regeneration area bounded by one layer from the bottom of the semicircular depression was observed and all the cortical bone layers recovered their thicknesses which was almost equivalent to those of the surrounding areas.

### (3) Quantitative analysis of bone mineral density in the X-ray microscopic image data obtained using the micro CT system

### At 2 weeks after the perforation:

Volume-rendering image (FIG. 8A) : Both in the control and diatomaceous earth groups, a blocking substance serving to block the semicircular depression which had a much higher radiolucency, i.e., considerably lower density, than the surrounding areas was observed.

Sagittally sectional image (FIG. 8B) : In the control rats, although the half part of the depression was filled with the low-density blocking substance, the bottom had a low density without closure in the perforation communicating with the medullary cavity and thus restoration at the bottom resulting from dense regenerated bone was not observed. In contrast, in the diatomaceous earth-administrated groups, restoration resulting from tightly dense regenerated bone was observed at the bottom. The volume of the blocking substance in the depression was smaller than that in the depression of the control rats. This was probably because the regeneration of the cortical bone has been already progressing more rapidly than in the control group and thus bone substitution during early-stage mineralization caused by the filling substance through calcification occurred more rapidly than in the control group. In the diatomaceous earth-administered groups, the presence of an area of a decreased density was recognized in the lamellar bone of the cortical bone in the vicinity of the perforation. This was probably because, as described above, at this stage, early mineralization in the wound and subsequent bone substitution resulting from calcification are promoted by the administration of diatomaceous earth, thereby absorption and dissolution of calcific component of bones as well as its addition to the wound in the vicinity became coordinately active.

### At 4 weeks of perforation (FIG. 8C):

Volume-rendering image: Although large differences were not observed, on the whole, the diatomaceous earth-administered groups had higher bone densities.

Sagittally sectional image (FIG. 8D) : In the control rats, although the semicircular depression became shallow, the thicknesses of all the cortical bone layers were still insufficient. The bone mineral density of the regenerated bone at the bottom was almost equivalent to those of the surrounding areas. On the other hand, in the diatomaceous earth-administered groups, the semilunar area of bone regeneration, bounded by one layer from the bottom of the semicircular depression, was observed and the cortical bone layers recovered their thicknesses which was almost equivalent to those of the surrounding areas. The bone density was similar to those of the surrounding areas. It was therefore concluded that the bone of the diatomaceous earth-administrate reel groups was recovered to the degree that it can bear loads in terms of structural strength as well.

### INDUSTRIAL APPLICABILITY

According to the present invention, hard-tissue regeneration promoters, pharmaceutical compositions, food compositions, foods, animal feeds, etc., useful for regeneration of impaired or defective hard tissues, which can be prepared in low production and labor costs.

## Claims

1. A hard-tissue regeneration promoter, comprising diatomaceous earth as an active ingredient.

2. The hard-tissue regeneration promoter of claim 1, wherein the hard tissue is a bone or a reparative dentin.

3. The hard-tissue regeneration promoter of claim 2, wherein the bone is an alveolar bone.

4. The hard-tissue regeneration promoter of any one of claims 1 to 3, wherein said promoter is administered beneath the periosteum.

5. The hard-tissue regeneration promoter of any one of claims 1 to 3, wherein said promoter is administered into a medullary cavity.

6. The hard-tissue regeneration promoter of any one of claims 1 to 3, wherein said promoter is administering orally.

7. A pharmaceutical composition, comprising diatomaceous earth as an active ingredient.

8. The pharmaceutical composition of claim 7, wherein said composition is an agent to be administered beneath the periosteum.

9. The pharmaceutical composition of claim 7, wherein said composition is an agent to be administered into a medullary cavity.

10. The pharmaceutical composition of claim 7, wherein said composition is an agent to be administered orally.

11. A food composition comprising diatomaceous earth.

12. A food comprising diatomaceous earth.

13. An animal feed comprising diatomaceous earth.
